# EUROPEAN PATENT APPLICATION

(11) **EP 2 372 364 A1**
(43) Date of publication of application: **05.10.2011**
(21) Application number: 10003562.5
(22) Date of filing: 31.03.2010
(51) Int. Cl.: G01N 33/74

(54) **Biomarker for pulmonary hypertension**

(71) Applicant: Medizinische Hochschule Hannover, 30625 Hannover (DE)
(72) Inventor: Kümpers, Philipp, 30171 Hannover (DE); Hoeper, Marius, 30625 Hannover (DE); Nickel, Nils, 30627 Hannover (DE)
(74) Representative: Kröncke, Rolf

(57) **Abstract**

The present invention relates to new methods for predicting the clinical outcome or determining the treatment course in a subject afflicted with pulmonary hypertension and for monitoring the severity and the progression of therapy of pulmonary hypertension in a subject. Moreover, the present invention relates to a method for stratification of the therapeutic regimen of a subject afflicted with pulmonary hypertension. Moreover, the present invention provides new biomarker for the diagnosis, identification or determination of the severity and clinical outcome of pulmonary hypertension. In particular, the present invention is based on the finding that determining the level or amount of angiopoietin-2, angiopoietin-1 or Tie2 in a sample of a subject is useful for conducting the above referenced methods.

## Description

The present invention relates to new methods for predicting the clinical outcome or determining the treatment course in a subject afflicted with pulmonary hypertension and for monitoring the severity and the progression of therapy of pulmonary hypertension in a subject. Moreover, the present invention relates to a method for stratification of the therapeutic regimen of a subject afflicted with pulmonary hypertension. Moreover, the present invention provides new biomarker for the diagnosis, identification or determination of the severity and clinical outcome of pulmonary hypertension. In particular, the present invention is based on the finding that determining the level or amount of angiopoietin-2, angiopoietin-1 or Tie2 in a sample of a subject is useful for conducting the above referenced methods.

### Background of the invention

Screening and monitoring assays are essential for the diagnosis and management of diseases or disorders. In particular, blood based remote samples for such applications have the advantage that it is convenient for a subject to provide a sample and the risk of side effects is extremely low. Therefore, compliance is improved in a test population.

Pulmonary hypertension (PH) is an increase in blood pressure in the pulmonary artery, pulmonary vein or pulmonary capillaries, together known as the lung vasculature, leading to shortness of breath, dizziness, fainting and other symptoms, all of which are exacerbated by exertion. Pulmonary hypertension can be a severe disease with a markedly decrease exercise tolerance and heart failure. At present, five different types of pulmonary hypertension may be identified: arterial, venous, hypoxic, thromboembolic or miscellaneous PH.

At present, a considerable procedure is required to confirm the presence of pulmonary hypertension and to exclude other possible diagnosis. These procedures include pulmonary function tests, blood tests, echocardiography, etc.

Pulmonary arterial hypertension is one type of pulmonary hypertension which is characterised by two further conditions of having a pulmonary arterial wedge pressure below 15 mm Hg (2000 Pa) and having a pulmonary vascular resistance (PVR) of greater than 3 Wood units (2.4 mN•s•cm⁻⁵). An overview about diagnosis and assessment of pulmonary arterial hypertension is provided in Badesch et. al., JACC, 2009, 54, 1, Suppl., S55-66. One form of pulmonary arterial hypertension (PAH) is the idiopathic pulmonary arterial hypertension (IPAH). The idiopathic pulmonary arterial hypertension is a devastating chronic disease caused by progressive pulmonary vascular remodeling, which results in right ventricular overload and failure if not treated effectively. Among the molecular mechanisms involved in the pathogenesis of IPAH, the angiopoietin-Tie2 ligand-receptor system has been recognized as a major signaling pathway that controls vascular remodeling and stabilization in a non-redundant manner.

A classification of pulmonary hypertension is provided with the Venice 2003 Device Stratification System identifying the five subgroups as identified above. WHO group 1 comprises the pulmonary arterial hypertension including idiopathic pulmonary arterial hypertension. The IPAH is typically not an accompanying disease or disorder of other diseases.

Fast and reliable diagnosis of pulmonary hypertension, like pulmonary arterial hypertension, in particular, IPAH, is necessary to allow therapy of the same. In particular, a more convenient approach is desired compared to the presently required measurement on the basis of echocardiography or measurements using a catheter. Typically, diagnosis of PAH requires right sided catheterisation.

Of note, the prognosis of IPAH without therapy is about 2 to 3 years after diagnosing the same.

Angiopoietins are angiogenic factors essential for vascular development and maturation. As circulating or matrix-bound molecules, angiopoietin-1 (Ang-1) and its antagonist angiopoietin-2 (Ang-2) bind to the extracellular domain of the tyrosine kinase receptor Tie2 which is almost exclusively expressed on endothelial cells. Produced by vascular smooth-muscle cells (SMCs) and precursor pericytes, Ang-1 stabilizes the development of newly formed blood vessels by recruiting mural cells and promotes quiescence and structural integrity of mature vessels. The importance of operational Ang-1/Tie2 signaling for developmental angiogenesis is illustrated by either Ang-1 ^{-/-} and Tie2 ^{-/-} knockout mice which die *in utero* owing to severe vascular remodeling defects causing perturbed vascular integrity. In the adult vasculature, constitutive Ang-1 expression and low-level Tie2 phosphorylation probably represent a control pathway to maintain vascular quiescence by antiapoptotic and anti-inflammatory effects, thus protecting the endothelium from excessive activation by cytokines and growth factors.

Ang-2 is expressed in endothelial cells, where it is stored in granules, the so-called Weibel-Palade bodies. The release of Ang-2 upon activation of the endothelium with for instance thrombin, histamine, or hypoxia disrupts the constitutive Ang-1/Tie2 signaling by preventing Ang-1 from binding to the receptor. Consequently, loss of Tie2 signaling destabilizes the endothelium and induces an angiogenic response in the presence of Vascular Endothelial Growth Factor (VEGF), whereas in the absence of VEGF, Ang-2 induces endothelial cell death and vessel regression. Hence, Ang-2 functions as a dynamic autocrine negative regulator of the quiescent resting endothelium.

Several lines of evidence suggest that aberrant activation of Tie2 is causally involved in PAH pathophysiology although the complex interaction between these angiogenic and anti-angiogenic molecules remains incompletely understood. Tie2 activation attenuates bone morphogenetic protein (BMP) signaling and increases the concurrent release of serotonin, a potent stimulator of SMC proliferation in human pulmonary artery endothelial cells (ECs). Both mechanisms have already been implicated in PAH pathophysiology (Du I. at al., N Engl. J. Med. 2003, 348, 500-509; Sullivan C.C., et al., 2003 PNAS, 100, 12331-12336). Further support for the causative role of Ang-1 in PAH comes from the fact that overexpression of Ang-1 in rodents results in a PAH-like phenotype Sullivan C.C., above. Consistently, overexpression of a soluble Tie2 ectodomain, which sequesters Ang-1, suppresses the pulmonary hypertension (PH) phenotype in Ang-1-induced PH (Kido M., et al., J Thora Cardiovasc Surg, 2005, 129, 268-276). On the other hand, Stewart et al. reported that operative Ang-Tie2 signaling protects from endothelial apoptosis, capillary rarefication, and finally the development of PH (Zhao Y.D., Circ. Res., 2003, 92, 984-991).

Accordingly, there is a need in the art for approaches that afford detection and allow determination of the treatment regimen of PH, in particular, PAH, like IPAH, having the added benefit of being cost effective, rapid and minimal invasive, preferably non-invasive. Approaches that determine the prognosis of PH, PAH or IPAH based on determining the severity of said deceases and determining the clinical outcome as well as allowing monitoring of treatment response are of additional value and can help identifying the therapeutic regimen as well as allowing prognosis of the course of disease for the individual patient.

There is also a need for allowing follow-up or monitoring the progression of PH, PAH, in particular, IPAH as well as for stratification of a subject afflicted with said diseases, e.g. according to the treatment response of said subject using biomarkers, preferably by a non-invasive and cost effective method or system allowing the same.

The present invention aims for providing new biomarkers particularly useful in the issues described above.

### Summary of the present invention

In a first aspect, the present invention relates to a method for diagnosing or identifying pulmonary hypertension (PH) in a subject comprising
a) Determining the level or amount of at least one of angiopoietin-2 (Ang-2), angiopoietin-1 (Ang-1) or Tie2 in a sample of the body fluid of the subject; and
b) Comparing the level or amount determined in step a) to a reference value reflecting the level or amount of at least one of angiopoietin-2 (Ang-2), angiopoietin-1 (Ang-1) or Tie2 in subjects not afflicted with PH,
wherein an increase in the level or amount relative to the reference value is indicative for pulmonary hypertension.

In a further aspect, the present invention relates to a method for the stratification of the therapeutic regimen of a subject afflicted with pulmonary hypertension, comprising:
a) Determining the level or amount of at least one of Ang-2, Ang-1 or Tie2 in a sample of said subject; and
b) Determining the susceptibility of said subject to a therapy based on the level or amount of Ang-2, Ang-1 or Tie2.

A further embodiment of the present invention relates to a method for monitoring the progression of therapy of pulmonary hypertension in a subject, comprising:
a) Detecting the level or amount of at least one of Ang-2, Ang-1 or Tie2 in a first sample from the subject at a first point in time;
b) Determining the level or amount of at least one of Ang-2, Ang-1 or Tie2 in a second sample from the subject at a second point in time; and
c) Comparing the level or amount of Ang-2, Ang-1 or Tie2 determined in step a) to the level or amount detected in step b) or to a reference value.

Another embodiment of the present invention relates to a method for predicting a clinical outcome, determining the severity or determining the treatment course in a subject afflicted with pulmonary hypertension, comprising:
a) Determining the amount of at least one of Ang-2, Ang-1 or Tie2 in at least one sample of the body fluid of said subject; and
b) Predicting the clinical outcome, determining the severity or determining the treatment course based on the amount of Ang-2, Ang-1 or Tie2 present in the sample.

That is, the present inventors recognised that independently each of Ang-2, Ang-1 and Tie2 present in the body fluid of a subject afflicted with PH, in particular with PAH, for example IPAH, represents a suitable biomarker for diagnosing or identifying said diseases or disorders as well as determining the severity of said diseases or disorders. In particular, it has been identified that an increased amount or level of either Ang-2, Ang-1 and/or Tie2 in a body fluid sample of a subject, in particular, in a blood sample, like plasma sample or serum sample, allows the diagnosis and determination of the treatment course as well as identification of the severity of subjects with PH, like PAH, in particular, with IPAH, as well as the stratification of a therapeutic regimen.

Moreover, the present invention relates to a biomarker for pulmonary hypertension, in particular, PAH, most preferably IPAH, which is at least one of Ang-2, Ang-1 or Tie2, preferably Ang-2.

Finally, the present invention provides a test kit for use in a method according to the present invention comprising means of determining the level or amount of at least one of Ang-2, Ang-1 or Tie2 on protein or mRNA level in a body fluid sample of a subject to be tested as well as instructions how to use the test kit. Preferably, said test kit is an ELISA.

### Brief description of the drawings

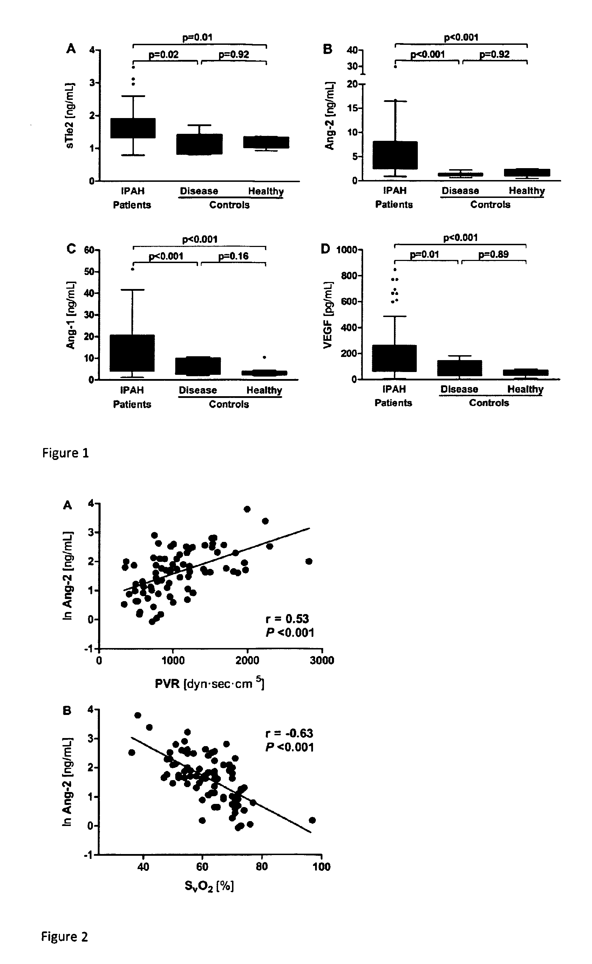

**Figure 1****: Angiogenic factors are elevated in IPAH.** Box plots showing plasma levels of Angiopoietin 1 (Ang-1, Fig.1A) and 2 (Ang-2, Fig.1B), soluble Tie-2 receptor (s-Tie-2, Fig.1 C) and vascular endothelial growth factor (VEGF, Fig.1D). The differences between patients with idiopathic pulmonary hypertension and control subjects were assessed by Mann-Whitney U Test. IPAH denote idiopathic pulmonary arterial hypertension. PAP denotes pulmonary arterial pressure. Ang-1 and Ang-2 were available from n=81 individuals with IPAH, n= 10 patients with mean pulmonary arterial pressure (PAPm) <25 mmHg (disease controls) and n= 14 apparently healthy individuals (healthy controls), s-Tie-2 and VEGF were available from n=60 individuals with IPAH, n= 10 individuals with PAPm<25 mmHg and n=14 apparently healthy individuals.
**Figure 2****: Circulating Ang-2 correlates with disease severity.** Scatter plots showing the relation of the natural logarithm of Angiopoietin-2 (In-Ang-2) to A) pulmonary vascular resistance (PVR), and B) mixed venous oxygen saturation (SvO₂) in the first patient cohort.

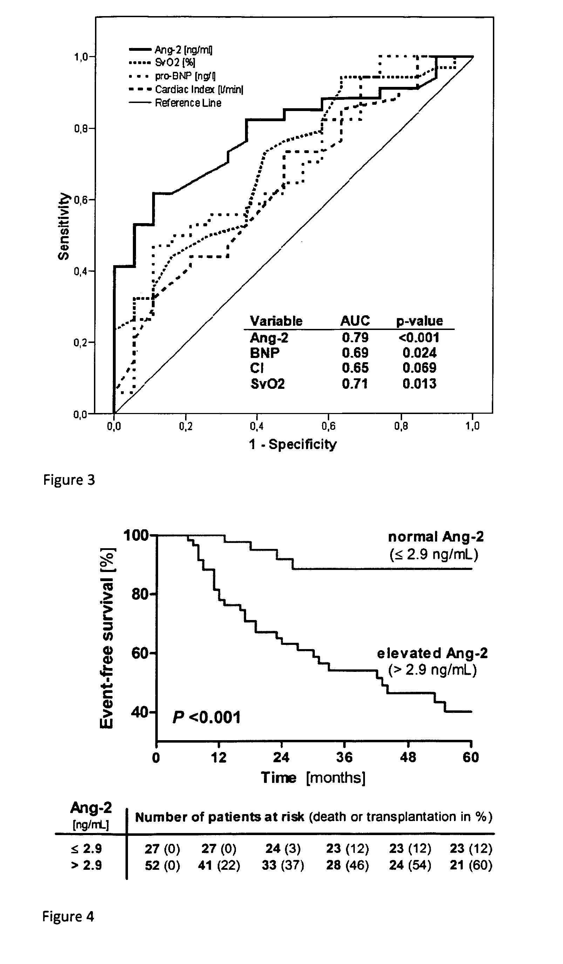

**Figure 3****: Ang-2 in the context of other markers of adverse prognosis.** Ang-2 Receiver-operator characteristic (ROC) curves showing the prognostic sensitivity and specificity of Angiopoietin-2 (Ang-2), NT-proBNP, cardiac index, and SvO₂ in the first patient cohort at baseline with regard to the composite endpoint at 3 years.
**Figure 4****: Elevated Ang-2 predicts poor outcome.** Kaplan-Meier curve showing the probability of event-free survival (death or transplantation) in the first patient cohort according to baseline Ang-2 levels above or below the previously defined reference value (2.9 ng/mL). The difference between the curves was determined by using a Log-Rank test.

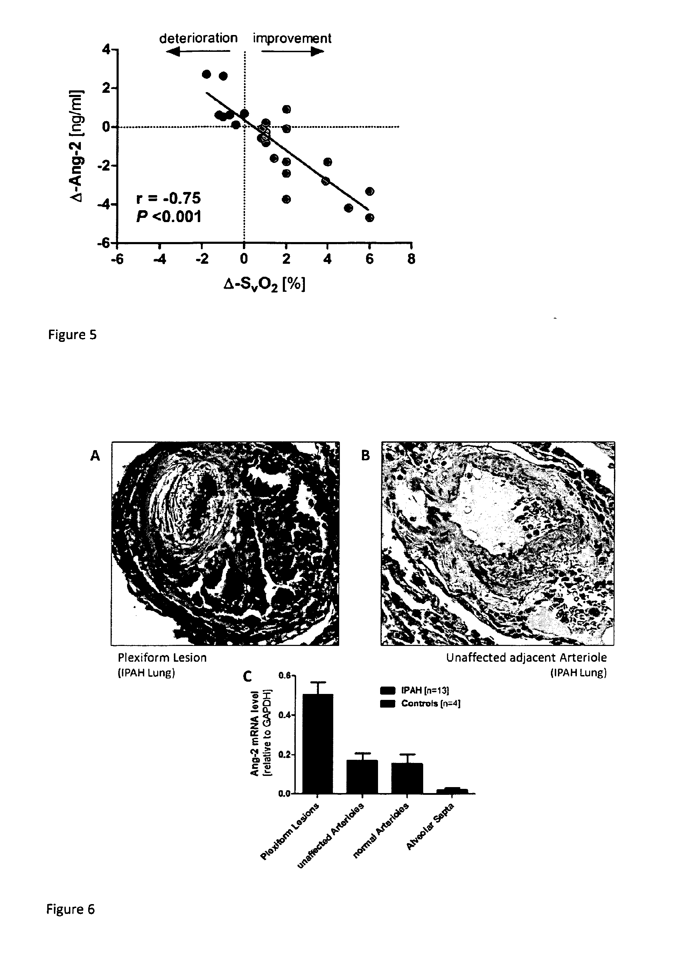

**Figure 5****: Changes of Ang-2 are closely related to treatment response.** Scatter plot showing the relation of changes in Ang-2 (Δ-Ang-2) to changes in SvO₂ (Δ-SvO₂) after 3 months in patients from the prospective cohort (n=25).
**Figure 6****: Compartments-specific Ang-2 expression in lung tissue samples from IPAH patients and brain-dead donors.** A) Ang-2 protein expression of a plexiform lesion in an IPAH lung detected by immunohistochemical staining staining. The prominent luminal endothelial cells show a strong positivity for Ang-2, while the interstitial fibrocytes and smooth muscle cells do not stain. Horse radish peroxidase, black. Original magnification x 400. B) Ang-2 protein expression of an unremodeled small pulmonary artery in an IPAH lung. Neither the regular endothelial cells, nor the smooth muscle cells of the vascular wall show positivity for Angiopoietin-2. Horse radish peroxidase, brown. Original magnification x400. C) Compartment-specific expression of Ang-2 mRNA levels assessed by quantitative RT-PCR (relative gene expression adjusted to GAPDH) in lung tissue from patients with IPAH (n=13) after laser-assisted microdissection. Lung tissue from brain-dead organ donors (n=4) served as control.

### Detailed description of the present invention

The present invention relates in a first aspect to a method for diagnosing or identifying pulmonary hypertension (PH) in a subject comprising:
a) Determining the level or amount of at least one of angiopoietin-2 (Ang-2), angiopoietin-1 (Ang-1) or Tie2 in a sample of the body fluid of the subject; and
b) Comparing the level or amount determined in step a) to a reference value, wherein an increase in the level or amount relative to the reference value is indicative for pulmonary hypertension.

That is, the present inventors recognised that depending on the level or amount of Ang-2, Ang-1 or Tie2 in a body fluid sample of a subject suspected to suffer from pulmonary hypertension, it is possible to diagnose or identify pulmonary hypertension in said subject based on an increase in the level or amount of said biomarkers Ang-2, Ang-1 or Tie2 relative to a reference value.

In particular, in multi variant analysis, elevated Ang-2 represents an independent risk factor of mortality in patients suffering from PH, like PAH, in particular, IPAH. Moreover, changes in Ang-2 after initiation of therapy correlated with changes in other independent parameters, like the mean arterial pressure, the PVR value or the mixed venous oxygen saturation (SvO₂). Hence, Ang-2 as well as Ang-1 or Tie2 present in the body fluid of the subject, in particular, circulating Ang-2, Ang-1 or Tie2, like plasma Ang-2, Ang-1 or Tie2, represents a promising new biomarker allowing to diagnose or identify sadi diseases, to determine disease severity and response to treatment as well as allowing for monitoring the progression of therapy and the stratification of therapeutic regimen in patients with PH, like PAH, and in particular patients with IPAH.

That is, e.g. the determination of pre-therapeutic serum Ang-2, Ang-1 or Tie2 levels allow prediction of the clinical outcome of patients suffering from PH treated with conventional therapeutics beside the fact that determining the pre-therapeutic serum Ang-2, Ang-1 or Tie2 levels allow determining the severity of the disease.

In the context of the present invention, the term "body fluid sample" or "sample of the body fluid" is a biological sample isolated from the subject which can include without being limited thereto, whole blood, serum, plasma, lymphatic fluids, ascetic fluids, interstitial fluids, cerebrospinal fluids, saliva, sputum, sweat, or any other secretion, excretion or other bodily fluids obtained from said individual. Preferably, the body fluid is whole blood, plasma, serum or urine, in particular, serum or plasma.

A "subject" in the context of the present invention is preferably a mammal. The mammal can be a human, non-human primate, mouse, rat, dog, cat, horse, or cow, but are not limited to these examples. A subject can be male or female. A subject can be one who has been previously diagnosed with or identified as suffering from or having PH, in particular PAH, like IPAH, and, optionally, but need not have already undergone treatment for said disease or disorder. A subject can also be one who has been diagnosed with or identified as suffering from PH, in particular PAH, like IPAH, but who show improvements in the disease as a result of receiving one or more treatments for said disease or disorder. Moreover, a subject may also be one who has not been previously diagnosed or identified as having a PH, in particular PAH, like IPAH. A subject can also be one who is suffering from or at risk of developing PH, in particular PAH, like IPAH.

The term "determining" as used herein refers to assessing the presence, absence, quantity, level or amount of either a given substance within a clinical or subject derived sample, including qualitative or quantitative concentration levels of substances or otherwise evaluating the values or categorisation of a subject's clinical parameter.

Pulmonary hypertension or "PH" according to the present invention refers to diseases or disorders e.g. identified and defined in Badesch D., et. al., JACC, vol. 54, no. 1, suppl. S 2009, S55-66.

In a further aspect, the present invention relates to a method for monitoring the progression of therapy of pulmonary hypertension in a subject, comprising:
a) Detecting the level or amount of at least one of Ang-2, Ang-1 or Tie2 in a first sample from the subject at a first point in time;
b) Determining the level or amount of at least one of Ang-2, Ang-1 or Tie2 in a second sample from the subject at a second point in time; and
c) Comparing the level or amount of Ang-2, Ang-1 or Tie2 determined in step a) to the level or amount detected in step b) or to a reference value.

Moreover, the present invention relates to a method for the stratification of the therapeutic regimen of a subject afflicted with pulmonary hypertension, comprising:
a) Determining the level or amount of at least one of Ang-2, Ang-1 or Tie2 in a sample of said subject; and
b) Determining the susceptibility of said subject to a therapy based on the level or amount of Ang-2, Ang-1 or Tie2.

In another embodiment, the present invention relates to a method for predicting a clinical outcome for determining the severity or for determining the treatment course in a subject afflicted with pulmonary hypertension, comprising:
a) Determining the amount of at least one of Ang-2, Ang-1 or Tie2 in at least one sample of the body fluid of said subject; and
b) Predicting the clinical outcome, determining the severity or determining the treatment course based on the amount of Ang-2, Ang-1 or Tie2 present in the sample.

It is preferred that the biomarker which level or amount is detected in the method according to the present invention is Ang-2. The present inventors recognised that the level or amount of Ang-2 represents a valuable tool, namely, a valuable biomarker for determining disease severity and response to treatment in patients with PH, like PAH, in particular IPAH. Moreover, it is recognised that the expression of Ang-2 protein and mRNA, Ang-1 protein and mRNA, or Tie2 protein or mRNA is exclusively upregulated in plexiform lesions from IPAH lung tissue samples.

It is preferred that the sample of the subject is a blood sample, in particular, a plasma sample or a serum sample. Alternatively, the sample of the subject is urine. In particular, in case of Ang-1, it is preferred that the sample is a plasma sample.

Moreover, it is preferred that the level or amount of at least one of Ang-2, Ang-1 or Tie2 is determined on the protein level. Of course, determination of nucleic acid level is also possible and within the scope of the present invention.

A person skilled in the art will appreciate that a number of different methods are useful for determining the level of the relevant proteins of the invention. In one embodiment, protocols for determining the level of protein use agents that bind to Ang-2, Ang-1 or Tie2, like antibodies, antibody fragments, phage display proteins, aptamers, affibodies, chemical lingands, peptides, and combinations thereof.

The term "antibody" as used herein is intended to include monoclonal antibodies, polyclonal antibodies, and chimeric antibodies. The antibody may be from recombinant sources and/or produced in transgenic animals. The term "antibody fragment" as used herein is intended to include Fab, Fab', F(ab')2, scFv, dsFv, ds-scFv, dimers, minibodies, diabodies, and multimers thereof and bispecific antibody fragments. Antibodies can be fragmented using conventional techniques. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, dsFv, ds-scFv, dimers, minibodies, diabodies, bispecific antibody fragments and other fragments can also be synthesized by recombinant techniques.

Antibodies having specificity for Ang-2, Ang-1 or Tie2 may be prepared by conventional methods. A mammal, (e.g. a mouse, hamster, or rabbit) can be immunized with an immunogenic form of the peptide which elicits an antibody response in the mammal. Techniques for conferring immunogenicity on a peptide include conjugation to carriers or other techniques well known in the art. For example, the peptide can be administered in the presence of adjuvant. The progress of immunization can be monitored by detection of antibody titers in plasma or serum. Standard ELISA or other immunoassay procedures can be used with the immunogen as antigen to assess the levels of antibodies. Following immunization, antisera can be obtained and, if desired, polyclonal antibodies isolated from the sera.

In a preferred embodiment, the antibodies having specificity for Ang-2 do not cross-react with Ang-1 or vice versa.

In one embodiment of the invention, the agents, such as antibodies, antibody fragments, phage display proteins, aptamers, affibodies, chemical ligands or peptides that bind to Ang-2, Ang-1 or Tie2, are labelled with a detectable marker.

The label is preferably capable of producing, either directly or indirectly, a detectable signal. For example, the label may be radio-opaque or a radioisotope, such as ³H, ¹⁴C, ³²P, ³⁵S, ¹²³I, ¹²⁵I or ¹³¹I; a fluorescent (fluorophore) or chemiluminescent (chromophore) compound, such as fluorescein isothiocyanate, rhodamine or luciferin; an enzyme, such as alkaline phosphatase, betagalactosidase or horseradish peroxidase; an imaging agent; magnetic or paramagnetic labels, or a metal ion.

In another embodiment, the detectable signal is detectable indirectly. For example, a labelled secondary antibody can be used to detect the protein of interest.

A person skilled in the art will appreciate that a number of other methods are useful to determine the levels of Ang-2, Ang-1 or Tie2 protein in a sample, including immunoassays such as Western blots, ELISA, and immunoprecipitation followed by SDS-PAGE immunocytochemistry. In addition, protein arrays (including microarrays) are useful.

Furthermore, in one embodiment of the invention, additional clinically relevant biomarkers are tested along with Ang-2, Ang-1 and/or Tie2, such as specific pathogen-associated antigens.

In one embodiment of the invention, Ang-2 protein levels and any additional markers of interest are determined using multiplex technology. This technology has the advantage of quantifying multiple proteins simultaneously in one sample. The advantages of this method include low sample volume, cost effectiveness and high throughput screening. Antibody-based multiplex kits are available from Linco (Millipore Corporation, MA), Bio-Rad Laboratories (Hercules, CA), Biosource (Montreal, Canada), and R&D Systems (Minneapolis, MN).

The invention also includes kits for use in predicting a clinical outcome or determining the treatment course in a subject with PH, in particular PAH, like IPAH, or for the stratification of a subject with PH, in particular PAH, like IPAH, typically with instructions for the use thereof. The detection agent may be an antibody, antibody fragment, a phage display protein, an aptamer, affibody, chemical ligand or a peptide. The kit may comprise more than one detection agent. In a further embodiment, the kit includes antibodies directed against Ang-2, Ang-1 or Tie2 optionally with one or more of a medium suitable for formation of an antigen-antibody complex, reagents for detection of the antigen-antibody complexes and instructions for the use thereof. In an additional embodiment, the invention relates to a composition comprising an anti-Ang-2, anti-Ang-1 or anti-Tie-2 antibody, optionally provided together in a container.

In a preferred embodiment, the level or amount of angiopoietin-2 is determined by immunological methods, in particular, by ELISA techniques.

That is, in a preferred embodiment, that the plasma or serum level of Ang-2, Ang-1 or Tie2 is determined by ELISA.

In the context of the present invention, the term "reference value" refers to an index value, a value derived from one or more PH, PAH, IPAH risk prediction algorithms or computed indices, a value derived from a subject with the same disease or disorder, or a value derived from the subject diagnosed with or identified as suffering from PH, in particular PAH, like IPAH. In particular, e.g. in case of the method for diagnosing or identifying the disease or disorder, the reference value is obtained from subjects not afflicted with the same PH, in particular PAH, like IPAH and, in addition, the reference value represents a range or index obtained from at least two samples collected from subjects not afflicted with PH, in particular PAH, like IPAH.

The increase in the level or amount of Ang-2, Ang-1 or Tie2 is for example at least 5%, at least 10%, at least 15%, at least 20%, at least 25% or at least 50% of the reference value or normal control level, preferably, the increase is at least 35%, at least 40% and, most preferably, at least 50%. For example, the increase is at least 2 fold, 3 fold, 4 fold or more.

The methods according to the present invention are particularly useful wherein the pulmonary hypertension is a pulmonary arterial hypertension. In particular, the methods according to the present invention relates to idiopathic pulmonary arterial hypertension, IPAH.

When monitoring the progression of a therapy, e.g. the response to treatments in a patient afflicted with pulmonary hypertension, like PAH, in particular, IPAH, it is preferred that the first sample is taken from the subject prior to be treated for the respective disease or disorder and the second sample is taken from the subject after being treated at least once for said disease or disorder.

Further, it is preferred that the amount of Ang-2 or Ang-1 is at least three fold higher in the subject afflicted with PH, in particular, PAH, like IPAH, compared to a healthy subject or reference value obtained from a subject not afflicted with the respective disease.

In another aspect, the present invention relates to a method for allowing stratification of the therapeutic regimen of said subject afflicted with the respective diseases or disorders. That is, by determining the level of Ang-2, Ang-1 or Tie2 for example in the plasma or serum of said subject, give the attending physician the possibility to determine and predict the usefulness of therapy based on conventional therapeutics for said respective diseases. As demonstrated herein, plasma or serum Ang-2 levels are associated with a more severe form of the respective disease and, in addition, as demonstrated herein, in case of success for therapy, Ang-2 levels decrease in the serum or plasma.

In clear contrast to the biomarkers according to the present invention, Ang-2, Ang-1 and Tie2, serum VEGF or other marker did not correlate with the severity and the identification of the respective diseases.

In a further aspect, the present invention relates to the use of at least one of Ang-2, Ang-1 or Tie2 as a biomarker for pulmonary hypertension, in particular PAH, most preferably IPAH.

The biomarkers according to the present invention allow to determining the disease severity, the outcome and response to treatment in subjects afflicted with the respective diseases as well as diagnosing and identifying said diseases. It is in particular preferred that the biomarker is Ang-2.

Finally, the present invention relates to a kit for use in a method according to the present invention for predicting a clinical outcome or determining the treatment cause in a subject afflicted with PH, in particular PAH, like IPAH, or for the stratification of a subject afflicted with PH, in particular PAH, like IPAH to determine whether said subject is susceptible to a treatment or for monitoring the progression or severity of PH, in particular PAH, like IPAH comprising means of determining the level or amount of at least one of Ang-2, Ang-1 or Tie2 on a protein or mRNA level in a body fluid sample of a subject to be tested according to the instructions on how to use said test kit.

In a preferred embodiment, the test kit is an ELISA determining the level of at least one of Ang-2, Ang-1 or Tie2, in particular of Ang-2, on protein level.

The above disclosure generally describes the present invention. A more complete understanding can be obtained by reference to the following specific examples of certain embodiments of the invention without being limited thereto.

### Methods

### Patients and Study design

In all patients, the diagnosis of IPAH (defined as PAP ≥ 25mmHg and capillary wedge pressure ≤ 15 mmHg) was based on standard criteria with confirmation by right heart catheterization and exclusion of other forms of pulmonary hypertension by various laboratory studies, echocardiography, pulmonary function testing, chest X-ray, ventilation-perfusion scanning, chest computed tomography angiography, and /or pulmonary angiography, if necessary. All catheter examinations were done for clinical reasons unrelated to this study. The study was performed in accordance with the declaration of Helsinki and approved by the institutional review board. All patients gave written, informed consent.

There were two patient groups; the first patient cohort consisted of 81 non-selected, treatment-naïve patients with IPAH referred to Hannover Medical School between 1999 and 2008 (Table 1). The second patient cohort included 25 consecutive patients with IPAH studied prospectively at Hannover Medical School between 2006 and 2009 (Table 5). In contrast to the first cohort, these patients had systematic follow-up right heart catheterizations at baseline and 3 months after the introduction of PAH-targeted therapy (see Supplementary data file 1 for details on PAH-targeted therapy).

Baseline blood samples were collected in both IPAH cohorts and disease controls at the time of the initial right heart catheterization before the initiation of any PAH-targeted therapy. In the prospective IPAH cohort, additional blood samples were obtained 3 months after the introduction of medical therapy at the time of follow-up right heart catheterizations.

### Follow-up and outcome definitions

Patients from the first cohort were followed by regular outpatient assessments for a median of 38 months (range 3 to 89 months). Forty-four patients reached the primary composite endpoint of death (n=41) or lung transplantation (n=3). Survival status was censored on April 30, 2009. Two patients were lost to follow-up. In the prospective cohort patients were followed up for 3 months after initiating therapy; follow-up ended with a second right heart catheterization.

### Controls

There were two control groups; the first one consisted of ten patients referred to Hannover Medical School between 2007 and 2009 with suspected PH that was eventually excluded by normal findings on right heart catheterization (mean pulmonary arterial pressure below 25 mmHg) referred to as *disease controls* (Table 1). After comprehensive workup, the following disorders were diagnosed: obesity hypoventilation syndrome, coronary heart disease, pulmonary lymphangioleiomyomatosis, chronic obstructive pulmonary disease (3x), paroxysmal supraventricular tachycardia, hepatic arteriovenous malformation with high-output heart failure, arterial septal defect, and patent foramen ovale with platypnoe-orthodeoxia syndrome. The second control group consisted of 14 apparently healthy volunteers without a history of any chronic disease (referred to as *healthy controls).*

### Blood sampling and laboratory analyses

All samples were collected into ethylenediaminetetraacetic acid (EDTA) tubes and were immediately placed on ice. Within 30 minutes of collection, samples were centrifuged at 3,000 g for 10 minutes, divided into aliquots and stored at -80°C. Creatinine and uric acid were measured using standard laboratory techniques. N-terminal fragment of the B-type natriuretic peptide (NT-pro BNP) was determined using a sandwich immunoassay on an Elecsys 2010 instrument with a detection limit of 20 ng/L (Roche Diagnostics, Mannheim, Germany). All measurements were performed by investigators blinded to patients' characteristics and outcome.

### Quantification of circulating angiogenic factors

Plasma Ang-1 and Ang-2 were measured by in-house Immunoluminometric Assay (ILMA) methodology as previously reported in detail in Kuempers P., et al., Crit Care 2008, 12, R147 and Lukasz A., et al., Crit Care 2008, 12, R94. The assays had detection limits of 0.12 ng/ml (Ang-1) and 0.2 ng/ml (Ang-2). Inter-assay and intra-assay imprecision was ≤8.8% and 3.7% for Ang-1 and was ≤4.6% and 5.2% for Ang-2, respectively.

Plasma VEGF (biologically active VEGF-A₁₂₁ and VEGF-A₁₆₅) and soluble Tie2 were measured using commercially available sandwich ELISA kits (R&D Systems) according to the manufacturer's instructions. All assays were performed in duplicate by investigators blinded to patients' characteristics and outcome.

### Lung tissue sampling and immunohistochemical staining

Tissue samples from lung explants were obtained in 13 additional patients with IPAH as well as from 4 brain-dead organ donors. Formalin fixed and paraffin embedded lung tissue sections were stained with Angiopoietin-2 antibody (dilution 1:15, goat anti-human Angiopoietin-2 Antibody, R&D System, Minneapolis, MN, USA; secondary anti-mouse-polymer-antibody (Reagent 3, ZytoChem Plus (HRP) Polymer Kit, Zytomed Systems, Berlin, Germany): chromogene substrate: DAB (DAB Substrate Kit High Contrast, Zytomed Systems, Berlin, Germany) and counterstained with Haemalaun (see Supplementary data file 2 for details).

### Microdissection and quantitative RT-PCR

Laser-assisted microdissection of distinct anatomical lung structures was performed as described. Ang-2 expression levels (RT-PCR) were analyzed in plexiform lesions from IPAH patients and compared relatively to those in the unaffected adjacent arterioles within the same sample, as well as to arterioles, arteries, and alveolar septa in lung samples from brain-dead donors (see Supplementary data file 2 for details).

### Statistical analysis

Data are presented as absolute numbers, percentages, means with corresponding standard deviations, or medians with corresponding 25^{th} and 75^{th} percentiles (IQR). Baseline characteristics of IPAH patients and controls subjects and the differences of angiogenic factors between IPAH patients and controls were compared using the two-sided Mann-Whitney-U test. Chi-Square analysis was used to compare gender. The relationship between the angiogenic factors and hemodynamic as well as 6 minute walk distance was investigated using Pearson's product-moment correlation. In all parametric tests, preliminary analysis and transformation were performed to ensure no violation of the assumption of normality, linearity and homoscedasticity. The differences in proportions in outcome events at years 1 to 4 in different strata of Ang-2 levels were judged by Fisher exact test. The distribution of the time-to-event variables were estimated using the Kaplan-Meier method with log-rank testing. To identify predictors of outcome, Cox's proportional hazards regression analysis was performed. All variables found to be statistically significant at a 10% level in the simple model were then included in a multiple model using stepwise forward elimination. To fulfill the assumptions needed for the analysis, logarithmic (In) transformation of NT-proBNP and Ang-2 was performed. For comparison of the prognostic values of Ang-2, uric acid, NT-proBNP, and selected hemodynamic parameters, receiver operating characteristic (ROC) curves were generated, and the areas under the curves (AUC) were calculated. The Wilcoxon signed rank test was used to compare changes in hemodynamic parameters, 6 minute walking distance, and angiogenic factors over time. Changes in these parameters in relation to changes in Ang-2 over time were assessed by Spearman rank correlation coefficients. All tests were two-sided and significance was accepted at p<0.05. Data analysis was performed using SPSS (SPSS Inc, Chicago, Illinois, USA). Figures were prepared using the GraphPad Prism (GraphPad Prism Software Inc, San Diego, California, USA).

### Patient's characteristics

The first (retrospective) patient cohort consisted of 81 patients (65% female) with a median (interquartile range) age of 54 (43-62) years. Control subjects (exclusion of pulmonary hypertension by right heart catheterization) were not different with respect to age, gender, and body mass index (BMI), but showed significantly better hemodynamics and functional capacity compared to IPAH patients. Demographic, clinical, and biochemical characteristics of patients and controls are summarized in Table 1.

### Plasma levels of angiogenic factors are elevated in PAH

Median Ang-1 was 3-fold higher in patients with IPAH compared to apparently healthy controls (14.8 (11.8 - 17.9) vs. 4.4 (1.8― 6.9) ng/mL; p < 0.001). The same was true for Ang-2 (4-fold over controls) (6.5 (5.1 - 7.7) vs. 1.6 (0.8 - 2.3) ng/mL; p = 0.001), sTie2 (1.7 (1.5 ― 1.8) vs. 1.2 (1.0 - 1.4) ng/mL; p < 0.01) and VEGF (232.4 (174.8 - 290.1) vs. 53.7 (30.6 - 76.7) ng/mL; p < 0.001) (Figure 1). No differences were detected between apparently healthy controls and patient controls (mean pulmonary arterial pressure below 25 mmHg).

### Only Ang-2, but not Ang-1, sTie2 or VEGF correlates with disease severity

Next the relationship of circulating angiogenic factors and disease severity was analysed. Only Ang-2, but not Ang-1, sTie2 or VEGF, showed a strong and consistent association with several clinicopathologic variables in the first patient cohort (Table 2). The relation of Ang-2 to pulmonary hemodynamics was illustrated by a close correlation with mean right atrial pressure (mRAP) (r = 0.53, p<0.001) and pulmonary vascular resistance (PVR) (r =0.6, p<0.001) (Figure 2 A). Moreover, Ang-2 correlated positively with New York Heart Association (NYHA) class (r= 0.47, p<0.001) and was inversely correlated with cardiac index (r = -0.53, p<0.001) and mixed venous oxygen saturation (SvO₂) (r = -0.63, p<0.001) (Figure 2 B), respectively. These findings were confirmed in the second (prospective) group (see below). Ang-1 solely correlated with VEGF (r = 0.75, p <0.001), whereas Ang-2 solely correlated with sTie 2 levels (r = 0.49, p < 0.001), respectively.

### Elevated circulating Ang-2 is associated with impaired hemodynamics, excess NT-pro BNP levels and unfavorable outcome

ROC curve analysis demonstrated that Ang-2 was a strong predictor of adverse outcome in the first patient cohort. Of the 81 patients in the retrospective group, 44 (54.3%) who died during follow-up had significantly higher levels of Ang-2 at baseline (7.7 (5.8 - 9.6) ng/mL), compared to patients who survived (3.7 (2.7 - 4.8) ng/mL; p < 0.001). The best Ang-2 cutoff level for predicting outcome at 3 years was 2.9 ng/mL (sensitivity 85% and specificity 58%) with an AUC of 0.79 (95% confidence interval [Cl], 0.67 to 0.91) (Figure 3). Fifty-two patients (64% of the first cohort) had Ang-2 levels above 2.9 ng/mL. These patients showed a more advanced disease stages as evidenced by higher NYHA class, higher mRAP and pulmonary arterial pressure (PAP), and a higher PVR compared to patients with Ang-2 values ≤ 2.9 ng/mL) (Table 3). Moreover, patients with Ang-2 >2.9 ng/mL had a lower cardiac index, lower mixed venous oxygen saturation, and more elevated concentrations of uric acid and NT-pro BNP. No significant differences were observed with regard to age, gender, 6 minute walking distance, and Ang-1 or VEGF levels (Table 3).

Survival rates at one year were 100% in patients with baseline Ang-2 ≤ 2.9 ng/mL compared to 78% in patients with Ang-2 > 2.9 ng/mL (p<0.001). The gap widened during follow-up: 92% vs. 63% after 2 years, 88% vs. 54% after 3 years, and 88% vs. 46% after 4 years, respectively (all p < 0.001) (Table 3). The corresponding Kaplan-Meier curves are shown in Figure 4 (Log Rank Test: p=0.001).

### Ang-2 in the context of other markers of adverse prognosis

To test whether circulating Ang-2 could serve as an independent predictor of survival, we performed univariate Cox proportional hazards analyses, incorporating several established markers of adverse outcome (Table 4). All variables found to be statistically significant at a 10% level in the univariate analysis (mean RAP, cardiac index, mean PVR, SvO₂, uric acid, NT-proBNP, and Ang-2) were concurrently subjected to forward stepwise multivariate Cox regression analysis. As a result, Ang-2 emerged as the only independent predictor of the composite endpoint (Table 4). Similar results were obtained in uni- and multivariate Cox proportional hazards analyses incorporating continuous instead of dichotomized variables (see Supplementary data file 3).

### Changes of Ang-2 are closely related to treatment response

The second cohort consisted of 25 patients (63% female) with a median age of 54 (43-62) years. As in the first cohort, all angiogenic factors were elevated compared to controls, but only Ang-2 correlated with RAP (r = 0.56, p = 0,002), cardiac index (r = -0.44, p = 0.02), PVR (r = 0.5, p = 0.01), and mixed venous oxygen saturation (SvO₂) (r = -0.67, p<0.001). Baseline characteristics are shown in Table 5. Three months after the initiation of PAH-targeted therapy, pulmonary hemodynamics had improved significantly, as evidenced by a significant decrease of PVR (p<0.005) (Table 5). Ang-2, but not Ang-1, sTie2, VEGF, or NT-proBNP changed significantly from baseline to follow-up in the prospective cohort (4.3 (3.2 -5.4) at baseline vs. 3.4 (2.6 - 4.3) ng/mL after 3 months of therapy; p = 0.04) (Table 5). Changes in Ang-2 during follow-up were significantly correlated with changes in 6 minute walking distance (r = -0.72; p <0.05) mean RAP (r = 0.6; p = 0.008), PVR (r = 0.51; p = 0.04), and were inversely related to changes in SvO₂ (r = -0.75; p < 0.001) (see Supplementary data file 4) (Figure 5). Changes of Ang-2 over time were not related to the therapeutic agent used (ANOVA with Bonferroni correction: p=0.195).

### Ang-2 is exclusively expressed in plexiform lesions from IPAH lung tissue samples

In order to clarify the cellular origin of elevated circulating Ang-2 the protein expression of Ang-2 in lung tissue from IPAH patients was studied. As a result, endothelial-specific Ang-2 protein expression was exclusively up-regulated in plexiform lesions (i.e. arterioles undergoing remodeling), but was not expressed in unaffected adjacent arterioles within the same IPAH lung tissue sample (Figure 6 A, B). Consistently, mRNA expression was significantly elevated in microdissected plexiform lesions compared to normal arterioles from both, IPAH patients (n=13, ANOVA with Bonferroni correction p<0.001) and brain-dead organ donors (n=4 p<0.001).

Herein, the first comprehensive study on circulating Ang-1, Ang-2, and sTie2 in IPAH is provided. The decisive results are: 1) Compared to healthy or disease controls, IPAH patients are characterized by an excess of circulating Ang-1, Ang-2, and sTie2 2) Of those, at least Ang-2 correlated further with disease severity; 3) After adjustment for hemodynamic and biochemical variables, Ang-2 emerged as an independent predictor of outcome; 4) Changes in Ang-2 after initiation of medical therapy were closely related to changes in mean RAP, PVR, and inversely related to changes in cardiac output and SvO₂; 5) Up-regulated Ang-2 protein and mRNA expression was an exclusive feature of plexiform lesions. These findings have implications for the role of angiopoietins in the pathogenesis of IPAH and shows that circulating levels of Ang-2 is useful as biomarker in this patient population.

### Angiopoietin-1

Evidence is provided for a robust elevation of plasma Ang-1 in two independent cohorts of patients with IPAH. These findings are in accordance with results from the group of Thistletwaite (Thistlewaite P.A., et al., J Thora Cardiovasc Surg, 2001, 122, 65-73), who showed that Ang-1 expression is confined to the cytoplasm of SMC within the wall of small pulmonary arteries and arterioles in patients with PAH, but cannot be detected in normal human lung samples. Although this has not been tested formally, it is conceivable to assume that SMC-derived Ang-1 might account for elevated Ang-1 plasma levels in the present patients. Stewart et al. above, however, did not detect any difference in Ang-1 expression or Tie2 activation in lung tissue from patients with PAH or controls, respectively. Of note, Ang-1 levels were not increased in patients with congestive heart failure. Thistletwaite, above and Du, above, detected a strong linear correlation of both, Ang-1 expression and activation (i.e. phosphorylation) of Tie2 in lung samples from PAH patients with the degree of PVR, irrespective of the cause of the disease (i.e. idiopathic PAH vs. other forms of PH).Elevated circulating Ang-1 represents a surrogate parameter for increased vascular SMC burden *per se,* although it does not correlate with PVR.

### Angiopoietin-2

The somewhat surprising finding of elevated Ang-2 levels in the plasma from IPAH patients is difficult to reconcile because little attention has been paid to Ang-2 after initial studies showed no significant differences in Ang-2 expression between lung tissues from PAH patients and healthy controls (Du, above). As a key destabilizing signal involved in initiating angiogenic remodeling, Ang-2 expression is tightly controlled. Hence, Ang-2 mRNA is almost undetectable in the quiescent vasculature and is detected only at those sites undergoing remodeling (for instance in sprouting tumor blood vessels). As a Weibel-Palade body-stored molecule, however, Ang-2 protein is rapidly released and induced upon various stimuli by a multitude of factors, including cytokines, thrombin, activated platelets and leucocytes, and changes in blood flow or oxygenation.

Excessive endothelial cell proliferation, along with concurrent neoangio-genesis, is a common pathological feature in PAH. Consistent with these findings, endothelial Ang-2 protein and mRNA expression was highly up-regulated in plexiform lesions, but entirely absent from unaffected adjacent arterioles within the same IPAH lung tissue sample. Moreover, circulating Ang-2 levels were closely associated with tissue hypoxia, as evidenced by a relatively tight linear correlation of Ang-2 with SvO₂. From a biological perspective it is entirely possible that circulating Ang-2 levels facilitates local remodeling in the pulmonary vessels.

Beyond its potential role as a facilitator/mediator, circulating Ang-2 as well as Ang-1 and Tie2 fulfill several prerequisites of a useful biomarker: it correlates relatively tightly with established hemodynamic markers of disease severity and seems to be an independent predictor of survival. Of note, Ang-2 changes with therapy and tightly reflects changes in PVR, RAP, and functional capacity (6 minute walking distance). It was previously shown that circulating angiopoietins (stable for 24h at room temperature and for at least 4 freeze-thaw cycles) can be readily quantified by immunoassay methodology with excellent intra- and interassay imprecision (Lukasz, above). Together, these findings indicate that Ang-2 beside Ang-1 and Tie2 is a valuable biomarker for non-invasive monitoring of treatment response in IPAH.

NTpro-BNP has been validated as a marker of right ventricular dysfunction in PAH. In the present study, Ang-2 was much closer related to hemodynamic impairment than NTpro-BNP both in the retrospective and in the prospective group of patients. Ang-2 did also perform better than NTpro-BNP in terms of predicting survival. However, further prospective studies are needed to compare the performance of these two biomarkers. Another biomarker that has recently been shown to be of prognostic importance in IPAH is growth-differentiation factor 15 (GDF-15), overexpression of which appears to be related to tissue hypoxia. Thus, Ang-2, NTpro-BNP and GDF-15 may reflect various aspects of the hemodynamic compromise in patients with IPAH and it may therefore be useful to evaluate panels of these biomarkers in future studies.

In summary, circulating Ang-2 and Ang-1 and Tie2 are associated with hemodynamic compromise and outcome of patients with IPAH and thus serve as promising new biomarker of diseases severity and response to treatment in patients with IPAH.

**Table 1: Baseline characteristics of the first cohort**

| | **IPAH** (n=81) | **PAPm <25mmHg** (n= 10) | **p-Value** |
|---|---|---|---|
| Age [years] | 54 (43-62) | 56.5 (46 - 67) | 0.45 |
| Female [%] | 65 | 57 | 0.58 |
| Body mass index [kg/m²] | 24.4 (22.1-27.2) | 25.7 (18.1 ― 33.5 | 0.52 |
| 6 minute walking distance [m] | 370 (297 ― 444) | - | - |
| NYHA class | 3.0± 0.5 | - | - |
| I/II [n, %] | 4 (4.3) | - | - |
| III [n, %] | 60 (74.5) | - | - |
| IV [n, %] | 17 (21.3) | - | - |
| mean RAP [mmHg] | 7.6 (6 ― 9) | 1.5 (1 ― 3) | **<0.001** |
| mean PAP [mmHg] | 54 (51 ― 58) | 20 (17 - 23) | **<0.001** |
| mean PCWP [mmHg] | 6 (6-7) | 4.6 (2.5 - 6.7) | 0.28 |
| Cardiac output [L/min] | 3.9 (3.6 ― 4.2) | 5.4 (3.7 ―7.1) | **0.001** |
| Cardiac index [L/min/m²] | 2.2 (2 - 2.4) | 2.9 (2.3 ― 3.4) | **0.007** |
| PVR [dyn·sec·cm⁻⁵] | 1.067 (960 ― 1.174) | 240 (168 ― 312) | **<0.001** |
| SvO₂ [%] | 64 (61 ― 66) | 75 (67 ―83) | **<0.001** |

Except for gender [%] and NYHA class [Mean ± SD], data are shown as median (interquartile range). Differences between patients and controls were calculated by using Mann-Whitney-U test for continues variables and chi-square test for nominal variables. Data on 6 minute walking distance were available from n=30 individuals, mean right atrial pressure (mean RAP, n=81); mean pulmonary arterial pressure (mean PAP, n=81); pulmonary capillary wedge pressure (PCWP, n=81); cardiac output and cardiac index (n=81); pulmonary vascular resistance (PVR, n=80); mixed venous oxygen saturation (SvO₂, n=81).

IPAH denotes idiopathic pulmonary aterial hypertension, PAPm denotes mean pulmonary arterial pressure, NYHA class denotes New York Heart Association classification of heart failure.

**Table 2: Relationship between angiogenic factors, hemodynamics and 6 minute walk test.**

| | **Ang-1** | | **Ang-2** | | **s-Tie-2** | | **VEGF** | |
|---|---|---|---|---|---|---|---|---|
| **Variables** | r | *p* | r | *p* | r | *p* | r | *p* |
| 6 minute walking distance [m] | -0.11 | *0.54* | -0.34 | *0.87* | -0.57 | *0.81* | -0.15 | *0.49* |
| NYHA class | 0.18 | *0.56* | **0.47** | ***0.001*** | 0.18 | *0.15* | 0.07 | *0.58* |
| mean RAP [mmHg] | -0.06 | *0.58* | **0.53** | **<0.001** | **0.39** | ***0.03*** | 0.03 | *0.83* |
| mean PAP [mmHg] | 0.02 | *0.87* | 0.25 | *0.04* | 0.22 | *0.15* | -0.1 | *0.52* |
| Cardiac output [L/min] | 0.15 | *0.19* | **-0.49** | ***<0.001*** | -0.23 | *0.11* | -0.13 | *0.39* |
| Cardiac index [L/min/m²] | 0.09 | *0.19* | **-0.53** | ***<0.001*** | -0.21 | *0.15* | -0.08 | *0.59* |
| PVR [dyn·sec·cm⁻⁵] | -0.05 | *0.24* | **0.6** | ***<0.001*** | **0.32** | ***0.03*** | 0.02 | *0.93* |
| SVO₂ [%] | 0.02 | *0.84* | **-0.63** | ***<0.001*** | -0.28 | *0.05* | 0.08 | *0.62* |
| Ang-1 | - | - | -0.13 | *0.3* | 0.11 | *0.42* | **-0.75** | ***<0.001*** |
| Ang-2 | -0.13 | *0.3* | - | - | **0.49** | ***<0.001*** | 0.35 | *0.81* |
| s-Tie-2 | 0.11 | *0.42* | 0.49 | *<0.001* | - | - | -0.13 | *0.36* |
| VEGF | -0.75 | *<0.001* | 0.35 | *0.81* | -0.13 | *0.36* | - | - |

Data are from the retrospective patient cohort. The relationship between the angiogenic factors Angiopoietin-1 (Ang-1) Angiopoietin-2 (Ang-2) soluble Tie-2 receptor (s-Tie), vascular endothelial growth factor (VEGF) and hemodynamic as well as 6 minute walk testing was investigated using Pearsons product-moment correlation. Preliminary analysis and transformation were performed to ensure no violation of the assumption of normality, linearity and homoscedasticity. Data on 6 minute walking distance were available from n=30 individuals, mean right atrial pressure (mean RAP, n=81); mean pulmonary arterial pressure (mean PAP, n=81); cardiac output and cardiac index (n=81); pulmonary vascular resistance (PVR, n=80); mixed venous oxygen saturation (SvO₂, n=81) Ang-1 (n=81), Ang-2 (n=81), s-Tie (n=60), VEGF (n=60).

NYHA class denotes New York Heart Association classification of heart failure.

**Table 3: Baseline characteristics in relation to plasma Ang-2 level**

| **Variables** | **Ang-2 ≤ 2.9 ng/ml (n=27)** | **Ang-2 >2.9 ng/ml (n=52)** | **p-value** |
|---|---|---|---|
| Age [years] | 55 (48 ― 62) | 49 (44 ― 55) | 0.144 |
| Female [%] | 69 | 65 | 0.41 |
| 6 minute walking distance [m] | 381 (300 ― 463) | 315 (242 ― 388) | 0.218 |
| NYHA class | 3.0 ± 0.5 | 3.4 ± 0.5 | **0.017** |
| mean RAP [mmHg] | 4.1 (2.4 ― 5.9) | 9.4 (7.3 ― 11.4) | **0.001** |
| mean PAP [mmHg] | 47 (41 ― 52) | 57 (50 ― 63) | **0.026** |
| Cardiac output [L/min] | 3.2 (2.8 ― 3.6) | 2.8 (2.2 ― 3.5) | **<0.001** |
| Cardiac index [L/min/m²] | 2.9 (2.2 ― 3.4) | 1.8 (1.6 ― 1.9) | **<0.001** |
| PVR [dyn·sec·cm⁻⁵] | 698 (541 ― 854) | 1,263 (1,106 ― 1,421) | **<0.001** |
| SvO₂ [%] | 71 (67 ― 76) | 57 (54 ― 60) | **<0.001** |
| Uric acid [mg/dl] | 323 (273 ― 372) | 502 (452 ― 552) | **<0.001** |
| NT-proBNP [ng/l] | 259 (130 ― 386) | 2,321 (1,410 ― 3,232) | **<0.001** |
| Ang-1 [ng/ml] | 13.7 (7.2 ― 20.1) | 21.2 (15.9 ― 26.3) | 0.067 |
| Ang-2 [ng/ml] | 2.0 (1.7 -2.3) | 9.2 (7.3 ―11.1) | **<0.001** |
| sTie-2 [ng/ml] | 1.4 (1.2 ― 1.6) | 1.9 (1.7 ― 2.1) | **<0.001** |
| VEGF [pg/ml] | 238(125-351) | 307 (227 ― 387) | 0.13 |
| Survival | | | |
| 12 Months | 100% | 78% | **<0.001** |
| 24 Months | 92% | 63% | **<0.001** |
| 36 Months | 88% | 54% | **<0.001** |
| 48 Months | 88% | 46% | **<0.001** |

Data are from the retrospective patient cohort. Except for gender, survival [%] and NYHA class [Mean ± SD], data are shown as median (interquartile range). Patients are divided by a Receiver-Operator-Characteristics (ROC)-curve optimized Ang-2 cut off of 2.9 ng/mL. Two patients with Ang-2 ≤ 2.9 ng/mL were lost to follow-up: n=27). Differences were calculated by using Mann-Whitney-U test for continued variables, fisher exact test for gender and survival comparison for 12, 24, 36 and 48 months. Data on 6 minute walking distance were available from n=30 individuals, mean right atrial pressure (mean RAP, n=81); mean pulmonary arterial pressure (mean PAP, n=81); cardiac output and cardiac index (n=81); pulmonary vascular resistance (PVR, n=80); mixed venous oxygen saturation (SvO₂, n=81).

Ang-1, angiopoietin-1; Ang-2, angiopoietins-2; s-Tie-2, soluble Tie-2-Receptor; VEGF, vascular endothelial growth factor; NT-proBNP, N-terminal fragment of the B-type natriuretic peptide, NYHA class denotes New York Heart Association classification of heart failure.

**Table 4:Outcome in Relation to Clinical and Biochemical Variables at Baseline.**

| | **Simple model** | | **Multiple model** | |
|---|---|---|---|---|
| | **HR (95% Cl)** | **P-value** | **HR (95% Cl)** | **P-value** |
| **Mean RAP** [per 1 mmHg ↑] | 1.05 (1.02 - 1.10) | 0.003 | | |
| **Cardiac index** [per 0.5 l/min/m² ↓] | 1.37 (1.09 - 1.72) | 0.003 | | |
| **PVR** [per 100 dyn·sec·cm⁻⁵ ↑] | 1.16 (1.08 - 1.24) | <0.001 | | |
| **SvO₂** [per 5% ↓] | 1.10(1.02-1.12) | 0.001 | | |
| **Uric acid** [per 100 µmol/L ↑] | 1.56 (1.27 - 1.96) | <0.001 | | |
| **In NT-proBNP** [per 1 SD ↑] | 2.10 (1.25 -3.30) | <0.001 | | |
| **In Ang-2** [per 1 SD ↑] | 2.24 (1.50 - 3.61) | <0.001 | 2.17 (1.34 - 3.72) | **0.004** |
| **In Ang-1** [per 1 SD ↑] | 1.12 (0.82 - 1.54) | 0.469 | | |
| **In sTie-2** [per 1 SD ↑] | 1.25 (0.84 - 1.87) | 0.269 | | |
| **In VEGF** [per 1 SD ↑] | 1.16 (0.77 - 1.74) | 0.552 | | |

Data are from the first patient cohort. Estimated hazard ratios (HR), 95% confidence intervals (Cl), and p-values were calculated by simple and stepwise forward Cox regression analyses. NT-proBNP Ang-2, s-Tie-2 and VEGF were not normally distributed and therefore In transformed; hazard ratios refer to 1 SD in the In scale in these variables.

RAP denotes right atrial pressure; PVR, pulmonary vascular resistance; SvO₂ mixed venous oxygen saturation. Ang-1, angiopoietin-1; s-Tie-2, soluble Tie-2-Receptor; VEGF, vascular endothelial growth factor; NT-proBNP, N-terminal fragment of the B-type natriuretic peptide.

**Table 5: Characteristics of the prospective cohort at baseline and after 3 months of follow-up**

| | **Baseline** (n=25) | **Follow up** (n=25) | **p-Value** |
|---|---|---|---|
| 6 minute walking distance [m] | 367 (304 ― 431) | 381 (329 ― 434) | 0.42 |
| mean RAP [mmHg] | 6.8 (4.5 ― 9.2) | 5.7 (3.2 ― 8.2) | 0.72 |
| mean PAP [mmHg] | 47 (42 ― 52) | 42 (38 ― 48) | 0.18 |
| Cardiac output [L/min] | 4.0 (3.5 ― 4.5) | 4.5 (3.7 ― 5.4) | **<0.001** |
| Cardiac index [L/min/M²] | 2.3 (2 ― 2.5) | 2.6 (2.2 ― 3) | **<0.001** |
| PVR [dyn·sec·cm⁻⁵] | 839 (677 ― 1,002) | 717 (512 ― 922) | **0.005** |
| SvO₂ [%] | 65 (60 ― 68) | 66 (64 ― 69) | 0.34 |
| NT-proBNP [ng/l] | 585 (422 ― 1,911) | 509 (506 ― 1,980) | 0.26 |
| Ang-1 [ng/ml] | 8.6 (5.2 ― 12) | 5.1 (2.8 ― 7.4) | 0.42 |
| Ang-2 [ng/ml] | 4.3 (3.2 ― 5.4) | 3.4 (2.6 ― 4.3) | **0.04** |
| sTie-2 [ng/ml] | 1.3 (1.1 ― 1.5) | 1.3 (1 ― 1.5) | 0.68 |
| VEGF [pg/ml] | 257 (63 ― 451) | 224 (52 ― 500) | 0.77 |

Data are from the prospective cohort. Data are shown as median (interquartile range). Changes from baseline to 3 months follow-up were assessed by Wilcoxon Signed Rank test. Data on 6 minute walking distance were available from n=18 individuals. Data on s-Tie-2 and VEGF were available for 10 individuals. F/u denotes follow-up;

RAP, right atrial pressure; PAP, pulmonary arterial pressure; PVR, pulmonary vascular resistance; SvO₂ mixed venous oxygen saturation. Ang-1, angiopoietin-1; s-Tie-2, soluble Tie-2-Receptor; VEGF, vascular endothelial growth factor; NT-proBNP, N-terminal fragment of the B-type natriuretic peptide.

### Supplementary data file 1

PAH-targeted therapy during follow-up in the first patient cohort

| Therapeutic agents [n (%)] | **IPAH** (n=81) | **PAPm <25mmHg** (n= 10) |
|---|---|---|
| Prostanoids | 33 (41.3) | 7 (28.9) |
| ERA | 10 (12.8) | 2 (10.5) |
| PDE-5 Inhibitors | 3 (3.7) | - |
| ERA + PDE-5 Inhibitors | 12 (14.7) | 4 (15.8) |
| Other combination therapies (≥ 2 agents) | 23 (27.5) | 12 (44.7) |

IPAH denotes idiopathic pulmonary aterial hypertension, PAPm denotes mean pulmonary arterial pressure, ERA denotes Endothelin receptor antagonists, PDE-5 denotes phosphodiesterase type 5.

### Supplementary data file 2

### Tissue sampling

Tissue samples from lung explants were obtained in 13 additional patients with IPAH as well as from 4 brain-dead organ donors. Formalin-fixed, paraffin-embedded lung tissue was retrieved from the archive of the Institute of Pathology at Hannover Medical School following the guidelines of the local ethics committee. Complex vascular lesions in PAH patients were diagnosed by two experienced pathologists according to up to date histopathological criteria.

### Immunohistochemical Staining

Three µm thick serial sections from formalin fixed and paraffin embedded (FFPE) lung tissue were cut, mounted on Superfrost Plus glass slides (Gerhard Menzel GmbH, Braunschweig, Germany) and dried at 60°C over night. Slides were deparaffinized, incubated for 10 minutes in 3 % hydrogen peroxide and rehydrated. For epitope retrieval, the samples were incubated in TRIS-EDTA buffer (10mM Tris base, 1mM EDTA solution, 0.05 % Tween 20, pH 9.0) for 40 min at 95 °C. Nonspecific binding sites were blocked for 5 min to reduce background staining (Reagent 1, ZytoChem Plus (HRP) Polymer Kit, Zytomed Systems, Berlin, Germany). The slides were incubated for one hour at room temperature with Angiopoietin-2 antibody (dilution 1:15, goat anti-human Angiopoietin-2 Antibody, R&D System, Minneapolis, MN, USA). Unbound primary antibody was removed by washing with TRIS buffer for 6 minutes. A linking-antibody (dilution 1:1000, AffiniPure mouse anti-goat IgG (H+L), Jackson Im-munoResearch Laboratories Inc., West Grove, PA, USA) was subsequently applied for 30 minutes. Excess antibody was again removed by washing with TRIS buffer. For signal intensification tissue sections were treated for 20 minutes with Reagent 2 (ZytoChem Plus (HRP) Kit, Zytomed Systems, Berlin, Germany) and again washed with TRIS. Afterwards, the slides were incubated for 30 minutes with a secondary anti-mouse-polymer-antibody (Reagent 3, ZytoChem Plus (HRP) Polymer Kit, Zytomed Systems, Berlin, Germany) and washed with TRIS buffer. DAB (DAB Substrate Kit High Contrast, Zytomed Systems, Berlin, Germany) was added as chromogene substrate. The slides were rinsed with distilled water and counterstained for 2 minutes with Haemalaun, washed with water and mounted.

### Microdissection of Plexiform lesions

FFPE tissue sections 5 µm thick were mounted on a poly-I-lysin-coated membrane fixed onto a metal frame. After standard deparaffinization and hemalaun staining, the CellCut Plus system (MMI Molecular Machines & Industries AG, Glattbrugg, Switzerland) was used for laser-assisted microdissection. Distinct anatomical lung structures (plexiform lesions, normal arteries, arterioles and alveolar septa) were isolated using a no-touch technique, essentially as described in the art. Approximately 850 cells were harvested from serial sections in each compartment. RNA was isolated by phenol-chloroform extraction as described in the art.

### Quantitative RT-PCR

Reversed transcription and quantitative RT-PCR was performed as described in the art. In brief, Real-time RT-PCR was performed on an ABI PRISM 7700 Sequence Detector (Applied Biosystems, Foster City, CA). Commercially available PCR primers and TaqMan probes to amplify Ang-2 were used (Applied Biosystems, Foster City, CA). Measurements were replicated three times. Data were evaluated by calculating relative expression levels using the [DELTA][DELTA] C_{T}-method. Ang-2 expression levels in plexiform lesions from IPAH patients were compared to those in the unaffected adjacent arterioles within the same sample, as well as to arterioles and alveolar septa in lung samples from brain-dead donors by ANOVA with Bonferroni correction.

### Supplementary data file 3

Outcome in Relation to continuous Clinical and Biochemical Variables at Baseline.

| | **Simple model** | | **Multiple model** | |
|---|---|---|---|---|
| | **HR (95% Cl)** | **P-value** | **HR (95% Cl)** | **P-value** |
| **Mean RAP** | 1.03 (1.02 ― 1.15) | 0.04 | | |
| **Cardiac index** | 1.62 (1.3 ― 3.1) | 0.001 | | |
| **PVR** | 1.36 (1.1 - 2.54) | <0.001 | | |
| **SvO₂** | 1.61 (1.22 - 2.12) | 0.001 | | |
| **Uric acid** | 1.12 (1.07 - 1.46) | <0.001 | | |
| **NT-proBNP** | 1.20 (1.01 -1.40) | <0.001 | | |
| **Ang-2** | 1.94 (1.50 - 2.51) | <0.001 | 1.25 (1.04 ― 2.12) | **0.03** |
| **Ang-1** | 1.01 (0.96 - 1.04) | 0.872 | | |
| **sTie-2** | 1.16 (0.62 - 2.13) | 0.644 | | |
| **VEGF** | 0.99 (0.63 - 1.02) | 0.54 | | |

Data are from the first patient cohort. Estimated hazard ratios (HR), 95% confidence intervals (Cl), and p-values were calculated by simple and stepwise forward Cox regression analyses. NT-proBNP Ang-2, s-Tie-2 and VEGF were not normally distributed and therefore In transformed; RAP denotes right atrial pressure; PVR, pulmonary vascular resistance; SvO₂ mixed venous oxygen saturation. Ang-1, angiopoietin-1; s-Tie-2, soluble Tie-2-Receptor; VEGF, vascular endothelial growth factor; NT-proBNP, N-terminal fragment of the B-type natriuretic peptide.

### Supplementary data file 4

Changes in 6 min walk distance, hemodynamic variables and biomarker levels from baseline to 3 months follow-up.

| **Δ Variable** | **Δ Ang-2 / Δ variable** (baseline vs. follow-up) | |
|---|---|---|
| | **r** | **p-value** |
| Δ 6 minute walking distance [m] | -0.72 | **<0.05** |
| Δ mean RAP [mmHg] | 0.60 | **0.008** |
| Δ mean PAP [mmHg] | -0.14 | 0.85 |
| Δ Cardiac output [L/min] | -0.29 | 0.21 |
| Δ Cardiac index [L/min/m²] | -0.32 | 0.14 |
| Δ PVR [dyn·sec·cm⁻⁵] | 0.51 | **0.04** |
| Δ SvO₂ [%] | -0.75 | <0.001 |
| Δ NT-proBNP | 0.36 | 0.39 |
| Δ ang-1 [ng/ml] | -0.16 | 0.47 |
| Δ Ang-2 [ng/ml] | - | - |
| Δ sTie-2 [ng/ml] | 0.15 | 0.74 |
| Δ VEGF | 0.37 | 0.33 |

Data are from the prospective patient cohort. Changes (Δ) in baseline variables over time in relation to changes in Ang-2 over time were assessed by Pearson correlation. Data on 6 minute walking distance were available from n=10 individuals, s-Tie-2 (n=9) and VEGF (n=9).

RAP denotes right atrial pressure; PAP, pulmonary arterial pressure; PVR, pulmonary vascular resistance; SvO₂ mixed venous oxygen saturation, Ang-1, angiopoietin-1; s-Tie-2, soluble Tie-2-Receptor; VEGF, vascular endothelial growth factor; NT-proBNP, N-terminal fragment of the B-type natriuretic peptide.

## Claims

1. A method for diagnosing or identifying pulmonary hypertension (PH) in a subject comprising:
a) Determining the level or amount of at least one of angiopoietin-2 (Ang-2), angiopoietin-1 (Ang-1) or Tie2 in a sample of the body fluid of the subject; and
b) Comparing the level or amount determined in step a) to a reference value reflecting the level or amount of at least one of angiopoietin-2 (Ang-2), angiopoietin-1 (Ang-1) or Tie2 in subjects not afflicted with PH,
wherein an increase in the level or amount relative to the reference value is indicative for pulmonary hypertension.

2. A method for the stratification of the therapeutic regimen of a subject afflicted with pulmonary hypertension, comprising:
a) Determining the level or amount of at least one of Ang-2, Ang-1 or Tie2 in a sample of said subject; and
b) Determining the susceptibility of said subject to a therapy based on the level or amount of Ang-2, Ang-1 or Tie2.

3. A method for monitoring the progression of therapy of pulmonary hypertension in a subject, comprising:
a) Detecting the level or amount of at least one of Ang-2, Ang-1 or Tie2 in a first sample from the subject at a first point in time;
b) Determining the level or amount of at least one of Ang-2, Ang-1 or Tie2 in a second sample from the subject at a second point in time; and
c) Comparing the level or amount of Ang-2, Ang-1 or Tie2 determined in step a) to the level or amount detected in step b) or to a reference value.

4. A method for predicting a clinical outcome, determining the severity or determining the treatment course in a subject afflicted with pulmonary hypertension, comprising:
a) Determining the amount of at least one of Ang-2, Ang-1 or Tie2 in at least one sample of the body fluid of said subject; and
b) Predicting the clinical outcome, determining the severity or determining the treatment course based on the amount of Ang-2, Ang-1 or Tie2 present in the sample.

5. The method according to any one of the preceding claims, wherein the sample of the subject is a blood sample, in particular, a plasma sample or a serum sample.

6. The method according to any one of the preceding claims, wherein the level or amount of at least one of Ang-2, Ang-1 or Tie2 is determined on the protein level.

7. The method according to claim 3, wherein the first sample is taken from the subject prior to be treated for PH and the second sample is taken from the subject after being treated for PH.

8. A method according to any one the preceding claims, wherein the PH is pulmonary arterial hypertension (PAH), preferably idiopathic pulmonary arterial hypertension (IPAH).

9. The method according to any one of the preceding claims, wherein the biomarker is Ang-2.

10. The method according to any one of the preceding claims, wherein the level or amount of at least one of Ang-2, Ang-1 or Tie2 is determined by immunological methods, in particular, by ELISA.

11. The method according to any one of the preceding claims, wherein the amount of Ang-1 or Ang-2 is at least three fold higher in the subject afflicted with PH, in particular, PAH, like IPAH compared to a healthy subject or a reference value obtained from a subject not afflicted with PH.

12. The use of at least one of Ang-2, Ang-1 or Tie2 as a biomarker for pulmonary hypertension, in particular, PAH, most preferably IPAH.

13. The biomarker according to claim 12, useful for determining the disease severity, outcome and response to treatment in subjects afflicted with PH, like PAH, in particular IPAH, which is Ang-2.

14. A test kit for use in a method according to any one of claims 1 to 11 for predicting a clinical outcome or determining the treatment course in a subject afflicted with PH, in particular PAH, like IPAH, or for the stratification of a subject afflicted with PH, in particular PAH, like IPAH to determine whether said subject is susceptible to a treatment, or for monitoring the progression or severity of PH, in particular PAH, like IPAH comprising means of determining the level or amount of at least one of Ang-2, Ang-1 or Tie2 on a protein or mRNA level in a body fluid sample of a subject to be tested and instructions on how to use said test kit.

15. The test kit according to claim 14, which is an ELISA.
